# NEUE EUROPÄISCHE PATENTSCHRIFT

(11) **EP 1 568 388 B2**
(45) Veröffentlichungstag und Bekanntmachung des Hinweises auf die Entscheidung über den Einspruch: **28.07.2010**
(45) Hinweis auf die Patenterteilung: 31.10.2007
(21) Anmeldenummer: 05010294.6
(22) Anmeldetag: 07.05.1999
(51) Int. Cl.: A61M 5/20, A61M 5/315, A61M 5/32

(54) **Autoinjektionsgerät**
Automatic injection device
Dispositif automatique d'injection

(30) Priorität: 15.05.1998 DE 19822031
(43) Veröffentlichungstag der Anmeldung: 31.08.2005
(62) Teilanmeldung aus: 99810407.9
(73) Patentinhaber: TecPharma Licensing AG, 3401 Burgdorf (CH)
(72) Erfinder: Kirchhofer, Fritz, 3454 Sumiswald (CH); Steck, Jürg, 3422 Kirchberg (DE); Hostettler, Peter, 3423 Ersigen (CH); Jost, Stephan, 3203 Mühleberg (CH)
(74) Vertreter: Schwabe - Sandmair - Marx

(56) Entgegenhaltungen:
- EP-A- 0 516 473
- EP-A- 0 824 923
- US-A- 5 320 609
- US-A- 5 391 151
- US-A- 5 643 214

## Beschreibung

Amoinjeknonsgeräte dienen zur Verabreichung von Produkten, insbesondere medizinisch oder kosmetisch wirksamen Flüssigkeiten. Dabei wird eine Injektionsnadel, durch die das Produkt verabreicht wird, nach Auslösen eines Antriebsmechanismus automatisch ein vorgegebenes Wegstück weit in ein Gewebe hineingestochen.

Ein Autoinjektionsgerät, wie die Erfindung es betrifft, umfaßt zumindest ein Gehäuse, ein vom Gehäuse verschiebbar aufgenommenes Behälmis, aus dem ein zu veräbreichendes Produkt durch Vorschieben eines Kolbens durch eine an einem Auslaß des Behältnisses angeordnete Nadel ausgeschüttet wird, und ein vom Gehäuse verschiebbar aufgenommenes Abtriebsglied einer Antriebseinrichnung, das bei einer Autoinjektion zum Einstechen der Nadel das Behältnis dem Gehäuse gegenüber in Vorschubrichnung des Kolbens bis zu einer vorgegebenen vorderen Position und zum Ausschütten des Produkts den Kolben im Behältnis vorschiebt. Sobald das Behältnis seine vordere Position bei einer Autoinjektion eingenommen hat, ist die Nadel um eine vorgegebene Wegstrecke zusammen mit dem Behältnis vorgeschoben worden, wobei mit der Vorgabe der Wegstrecke auch im wesentlichen die Eindringtiefe der Nadel vorgegeben ist.

Derart ausgebildete Autoinjektionsgeräte sind aus der US-PS 159,192, der EP 0 516 473 B1 und der US-PS 5,643,214 bekannt.

Bei den bekannten Autoinjektionsgeräten erfolgt der Vorschub des Behältnisses mit der daran angebrachten Nadel durch Andruck des Abtriebsglieds auf den Kolben.

Aus der EP 0 824 923 A1 ist ein Autoinjektionsgerät bekannt, bei dem der Vorschub des Behältnisses relativ zu dem Gehäuse und der Vorschub des Kolbens im Behältnis voneinander entkoppelt sind. Die Entkopplung wird durch Lösen eines Klinkeneingriffs bewirkt.

Die Erfindung hat es sich zur Aufgabe gemacht, ein Autoinjektionsgerät mit einer mechanisch einfachen Entkopplung der Vorschubbewegung des Behältnisses und der Vorschubbewegung des Kolbens im Behältnis zu schaffen, die einen weicheren Übergang zwischen den beiden Vorschubbewegungen ermöglicht.

Nach der Erfindung ist das Abtriebsglied bei einem Autoinjektionsgerät der vorbeschriebenen Art bis zum Erreichen der vorderen Position des Behältnisses vom Kolben entkoppelt und wird bei Erreichen der vorderen Position des Behältnisses von dem Behältnis entkoppelt und zum Verschieben des Kolbens im Behältnis mit dem Kolben gekoppelt. Das Vorschieben des Behältnisses zum Einstechen der Nadel wird nicht mittels des Kolbens bewirkt, d.h. es besteht während dieser Bewegungsphase keine Antriebsverbindung zwischen dem Abtriebsglied und dem Kolben. Hierdurch wird sicher ausgeschlossen, daß bereits während des Vorschiebens des Behältnisses Produkt durch eine etwaige, wenn auch kleine, Vorschubbewegung des Kolbens im Behältnis, d.h. relativ zum Behältnis, ausgeschüttet wird. Indem in der vorderen Position des Behältnisses das Abtriebsglied vom Behältnis entkoppelt und erst jetzt mit dem Kolben gekoppelt wird, wird der Kolben frühestens dann im Behältnis vorgeschoben, wenn die Nadel bis in die gewünschte Tiefe in das Gewebe eingestochen worden ist. Die antriebsseitige Trennung der Vorschubbewegung des Behältnisses und der Vorschubbewegung des Kolbens im Behältnis erhöht die Genauigkeit der Dosierung, da ein Andruck auf den Kolben nicht bereits während des Vorschiebens des Behältnisses ausgeübt wird und dadurch Produkt vorzeitig ausgeschüttet werden kann.

Das Abtriebsglied kann zum Vorschieben des Behältnisses unmittelbar auf das Behältnis wirken.

Vorzugsweise ist ein Übertragungsglied vorgesehen, das von dem Abtriebsglied bei einem Vorschieben mitgenommen wird, dabei auf das Behältnis und/oder einen Behältnishalter wirkt und so das Behältnis vorschiebt. Das Übertragungsglied kann auch ein Behältnishalter sein, wird vorteilhafterweise jedoch als davon separates Teil ausgebildet.

Zwischen dem Abtriebsglied und dem Übertragungsglied besteht die lösbare Kopplung. Nach dem Lösen der Kopplung in der vorderen Behältnisposition bewirkt ein weiteres Vorschieben des Abtriebsglieds, nämlich zum Vorschieben des Kolbens, kein weiteres Vorschieben des Übertragungsglieds mehr. Das Abtriebsglied ist jetzt zumindest in Bezug auf seine eigene Vorschubbewegung vom Übertragungsglied frei. Es drückt nur noch gegen den Kolben und schiebt diesen im Behältnis dadurch vor. Vorzugsweise ist das Übertragungsglied vom Gehäuse verschiebbar in und gegen die Vorschubrichtung des Kolbens aufgenommen.

Nach der Erfindung schließt die Kopplung zwischen dem Abtriebsglied und dem Behältnis eine kraftschlüssige Verbindung zwischen dem Abtriebsglied und dem Behältnis bzw. dem Übertragungsglied zumindest mit ein.

Vorzugsweise wird eine form- und kraftschlüssig wirkende Kopplung zum Koppeln und Entkoppeln des Abtriebsglieds mit und von dem Behältnis sowie mit und von dem Kolben verwendet. Auch in dieser Ausführungsform ist die Kopplung vorzugsweise zwischen dem Abtriebsglied und einem Übertragungsglied ausgebildet, obgleich auch eine Kopplung unmittelbar mit dem Behältnis denkbar wäre.

Die Kopplung wird vorzugsweise durch eine Schnappverbindung gebildet. Beim Vorschieben des Abtriebsglieds drückt ein Schnapper gegen eine Andruckfläche und schiebt dadurch das Behältnis vor. Wenn das Behältnis seine vordere Position erreicht hat, in der es dem Gehäuse gegenüber auf Anschlag liegt, wird die Schnappverbindung durch die weiterhin auf das Abtriebsglied wirkende Antriebskraft gelöst. Das Abtriebsglied wird mit dem Kolben gekoppelt und frei vom Behältnis bzw. frei vom Übertragungsglied weiter vorgeschoben. Ein leichter Stoß bei Erreichen der vorderen Behältnisposition kann durchaus unterstützend wünschenswert sein.

Zumindest eines der die Kopplung bildenden Mittel ist nachgiebig ausgebildet, vorzugsweise elastisch nachgiebig. Ein oder mehrere Schnapper können unmittelbar am Abtriebsglied ausgebildet sein, das damit gegen eine Andruckfläche des Behältnisses oder des Übertragungsglieds drückt, falls ein solches verwendet wird. Solche Schnapper können auch am Übertragungsglied vorgesehen sein.

Besonders bevorzugt wird zur Ausbildung der Kopplung ein drittes Kopphmgsmittel mit einbezogen, das dann allein nachgiebig, vorzugsweise elastisch nachgiebig, ausgebildet ist. Vorteilhafterweise ist das dritte Kopplungsmittel ein nachgiebiger Ring, insbesondere ein Federring, der zwischen zwei einander gegenüberliegenden Flächen des Abtriebs- und des Übertragungsglieds eingeklemmt ist, und eine dieser beiden Andruckflächen überschiebt, sobald das Behältnis seine vordere Position erreicht hat.

Die Erfindung kann mit Vorteil auch bei Autoinjektionsgeräten Verwendung finden, bei denen das Behältnis durch eine sogenannte Mehrkammerampulle gebildet wird. Durch solche Mehrkammerampullen wird das zu verabreichende Produkt erst durch Mischung von Inhalten der mehreren voneinander separierten Kammern beim Zusammensetzen des Geräts erhalten. Jede der hintereinander angeordneten Kammern wird rückwärtig mittels eines im Behältnis verschiebbaren Kolbens abgeschlossen. Zum Durchmischen wird ein Mischglied gegen den hintersten Kolben gedrückt und schiebt bei seinem Vorschieben im Behältnis jeden der Kolben nach vorne bis zum vordersten Kolben. Beim Vorschieben wird bzw. werden Verbindungen zur jeweils benachbart vorderen Kammer hergestellt, so daß ein Inhalt der hinteren Kammer jeweils in die benachbart davorliegende Kammer verdrängt wird.

Nach der Erfindung bildet das an sich bekannte Mischglied, das insbesondere ein Mischrohr ist, nun gleichzeitig auch das Übertragungsglied zum Vorschieben des Behältnisses. Dem Mischglied wird erfindungsgemäß eine Doppelfunktion zugewiesen. Konstruktiv wird dies vorzugsweise dadurch erreicht, daß das Mischglied einen radial nach außen abragenden Steg, vorzugsweise in Form einer umlaufenden Schulter, aufweist, mit dem es gegen die hintere Stirnfläche des Behältnisses oder, falls dies bevorzugt wird, gegen eine rückwärtigen Andruckfläche eines Behältnishalters drückt, der bei seiner mittels des Mischglieds erzwungenen Vorwärtsbewegung das Behältnis mitnimmt. Auch die vordere Position des Behältnisses wird vorzugsweise durch Anschlag eines solches Behältnishalters gegen das Gehäuse definiert.

Das Gehäuse kann sowohl das Behältnis als auch die gesamte Antriebseinrichtung umhüllen, vorzugsweise die Form einer Hülse aufweisen, dient jedoch in seiner allgemeinsten Ausbildung lediglich als Basisteil, gegenüber dem die Verschiebebewegung des Behältnisses und die Verschiebebewegung des Abtriebsglieds der Antriebseinrichtung erfolgen und ist daher nicht ausschließlich, obgleich bevorzugt, als umhüllendes Gehäuse zu verstehen.

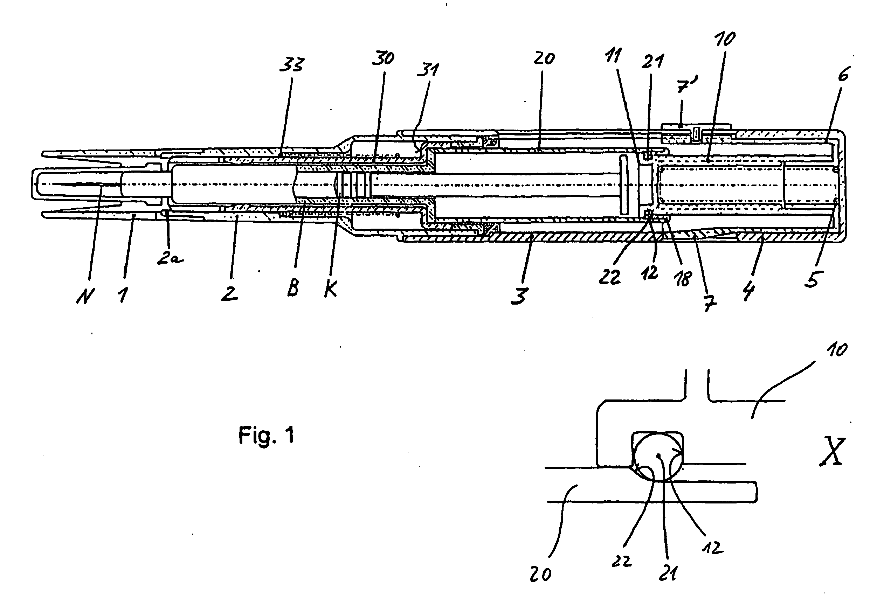

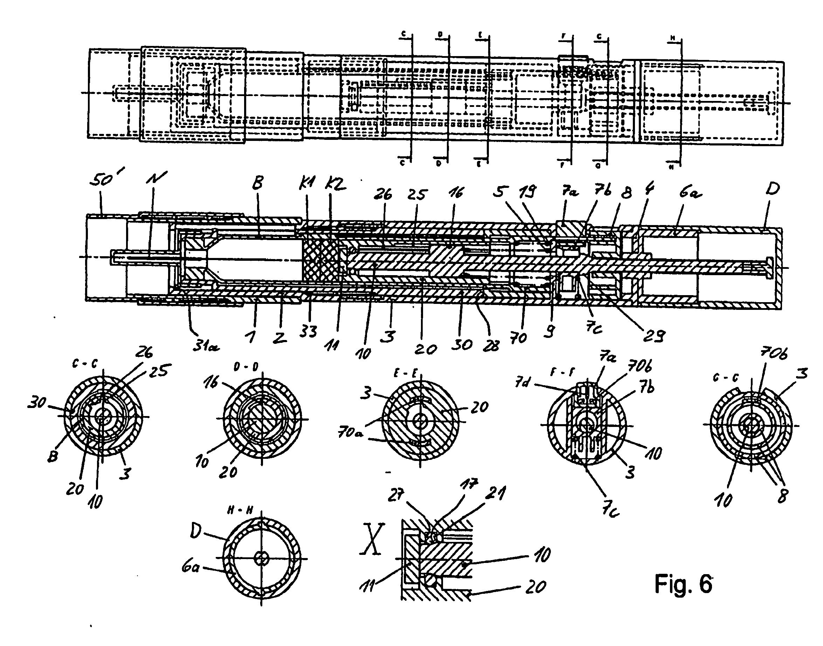

Ausführungsbeispiele der Erfindung werden nachfolgend anhand der Figuren 1 und 6 erläutert. Es zeigen:
- Fig. 1: ein Autoinjektionsgerät mit form- und kraftschlüssiger Kopplung,
- Fig. 2: ein Autoinjektionsgerät mit formschlüssiger Kopplung,
- Fig. 3: das Übertragungsglied des Geräts nach Fig. 2,
- Fig. 4, 5: die Sperre für die Nadelschutzhülse des Geräts nach Fig. 2 und
- Fig. 6: ein Autoinjektionsgerät mit einer Zweikammerampulle und einem als Übertragungsglied ausgebildeten Mischrohr.

Fig. 1 zeigt ein Autoinjektionsgerät in Form eines Injektionspens im Längsschnitt.

Der Pen weist ein hohlzylindrisches, im Ausführungsbeispiel kreiszylindrisches Gehäuse auf mit einem Nadelschutz 1 an seinem vorderen Ende, einer daran anschließenden vorderen Gehäusehülse 2 und einer sich daran anschließenden hinteren Gehäusehülse 3 mit einer diese stirnseitig abschließenden Gehäusekappe 4. Die vordere Gehäusehülse 2 umschließt ein zylindrisches Behältnis B, das seinerseits in einem Behältnishalter 30 koaxial zu einer Mittellängsachse des Gehäuses zentriert gehalten wird. Der Behältnishalter 30 ist ebenfalls hülsenförmig und läuft nach vorne in Zentrierzungen aus, die gegen den Umfangsrand des Behältnisses B drücken und dieses in der vorderen Gehäusehülse 2 zentrieren. Der Behält nishalter 30 ist entlang der Mittellängsachse des Gehäuses hin und her gleitverschiebbar. Beim Vorschieben des Behältnishalters 30 wird das darin gehaltene Behältnis B mit verschoben. Dabei wird eine an einem vorderen Ende des Behältnisses B an einem Auslaß angeordnete Injektionsnadel N nach vom geschoben und im Zuge einer Autoinjektion in und unter die Haut eines Patienten gestochen.

Im Behältnis B ist ein Kolben K entlang der Mittellängsachse des Behältnisses B verschiebbar. Durch Vorschieben des Kolbens K wird ein im Behältnis B enthaltenes Produkt, im Ausführungsbeispiel eine Medikamentflüssigkeit, beispielsweise Insulin, durch den Auslaß in und durch die Injektionsnadel N hindurch zum Ausschütten des Produkts verdrängt. Vom Kolben K ragt rückwärtig eine Kolbenstange über einen hinteren Behältnisrand hinaus ab.

Das Behältnis B ist entlang seines rückwärtigen Randes flanschartig verbreitert. Der Behältnishalter 30 ist an seinem rückwärtigen Ende entsprechend ebenfalls verbreitert und mit verbreitertem Querschnitt ein Stück weit über das Behältnis hinaus hülsenförmig fortgeführt. Das Behältnis B liegt im eingebauten Zustand mit seiner rückwärtigen Verbreiterung an der dieser Verbreiterung zugewandten Schulterfläche des Behältnishalters 30. Eine äußere Mantelfläche des Behältnishalters 30 ist von einer als Rückstellelement 33 dienenden Druckfeder umgeben, die mit einem vorderen Ende an einer Schulterfläche der vorderen Gehäusehälfte 2 anliegt und von dort nicht ganz bis zu einer Gegenfläche 31 ragt, die durch die Verbreiterung des Behältnishalters 30 am Behältnishalter 30 gebildet wird und der vorgenannten Schulterfläche der vorderen Gehäusehülse 2 zugewandt ist. Beim Vorschieben des Behältnishalters 30 und damit des Behältnisses B wird die Druckfeder 33 zwischen diesen beiden einander zugewandten Flächen zusammengedrückt und federt dadurch die Vorschubbewegung beim Einstechen der Nadel N ab. Die Fläche 31 stößt beim Vorschieben gegen eine Schulterfläche, die im Bereich einer stufenförmigen Verbreiterung der vorderen Gehäusehülse 2 gebildet wird. Der Anschlag der Schulterfläche 31 des Behältnishalters 30 definiert eine vordere Position des Behältnisses B und damit diejenige Länge, mit der die Nadel N in der vorderen Position des Behältnisses B über den vorderen Rand des Gehäuses hinaussteht, wodurch gleichzeitig auch, korrektes Aufsetzen des Pens auf die Haut vorausgesetzt, die Einstichtiefe der Nadel N vorgegeben ist.

Eine Antriebseinrichtung zum Vorschieben des Behältnisses B und zum Vorschieben des Kolbens K im Behältnis B wird durch ein als Druckfeder ausgebildetes Antriebselement 5 und ein Abtriebsglied 10 gebildet. Der Antrieb könnte auch mittels eines Druckmediums erfolgen. Das Abtriebsglied 10 ist im wesentlichen hülsenförmig mit einem rückwärtig offenen Hülsentopf, der durch einen Boden abgeschlossen wird. An seinem vorderen Ende ist das Abtriebsglied 10 auf die Kolbenstange zu über den genannten Boden hinaus ein vergleichsweise kurzes Stück weit hülsenförmig fortgesetzt. Die Fortsetzung bildet einen Stempel 11 zum Andrücken gegen die Kolbenstange und Vorschieben des Kolbens K im Behältnis B.

Im dargestellten Ausgangszustand des Injektionspens besteht kein unmittelbarer Andruck des Stempels 11 gegen die Kolbenstange. Es verbleibt im Ausgangszustand vielmehr ein kleiner Freiraum zwischen dem Stempel 11 und der rückwärtigen Stirnfläche der Kolbenstange. Im dargestellten Ausgangszustand des Pens, in dem das Behältnis B sich in seiner hinteren Verschiebeposition befindet, ist das Abtriebsglied 10 mit dem Behältnis B gekoppelt. Unmittelbar ist das Abtriebsglied 10 mit einem Übertragungsglied 20 gekoppelt, das dann unmittelbar auf das Behältnis B wirkt. Ebenso könnte das Übertragungsglied 20 auch unmittelbar auf den Behältnishalter 30 wirken, wobei dann zwischen dem Behältnishalter 30 und dem Behältnis B Mitnehmer vorzusehen wären. Grundsätzlich könnte auch das Abtriebsglied unmittelbar auf ein Behältnis oder einen Behältnishalter drücken, um die Nadel vorzuschieben.

Das Übertragungsglied 20 wird durch einen Hülsenkörper gebildet, in dessen hinteren Bereich durch Querschnittverengung eine umlaufende Schulter 22 ausgebildet ist, die als vordere Andruckfläche für einen federelastischen Ring 21 dient. Der federelastische Ring 21 ist in einer an der äußeren Mantelfläche des Abtriebsglieds 10 im Bereich des Stempels 11 umlaufenden Nut dem Abtriebsglied 10 gegenüber verschiebegesichert gehalten. Der Ring 21 kann als geschlossener weichelastischer Ring ausgebildet sein, vorzugsweise besteht er jedoch aus einem harten Material wie Kunststoff oder Metall, und ist an einer Stelle durchbrochen, so daß die dort aufeinander zu weisenden Enden des Rings 21 ein Stück weit aufeinander zu bewegt werden können und so der Durchmesser des Rings verringert werden kann. Der nachgiebige Ring 21, die Schulter 22 und die der Schulter 22 zugewandte Wandung 12 der Nut bilden eine auf Form- und Kraftschluß beruhende, lösbare Kopplung zwischen dem Abtriebsglied 10 und dem Übertragungsglied 20. Der Ring 21 als Andruck- und Kopplungsmittel könnte auch als Wulst am Abtriebsglied 10 oder Übertragungsglied 20 ausgebildet sein, das dann nach vom in abbiegbaren Zungen auslaufen würde.

Das Abtriebsglied 10 wird gegen den Druck des Antriebselements 5 in der in Fig. 1 dargestellten Ausgangslage von einer Halte- und Auslösehülse 6 mittels einer an der Halte- und Auslösehülse 6 ausgebildeten Auslöselasche 7 gehalten. Die Verbindung wird durch Einwärtsdrücken der Auslöselasche 7 gelöst.

Zum Injizieren des Produkts wird der Pen nach Abziehen einer Nadelschutzkappe mit dem vorderen Rand des Nadelschutzes 1 mit leichtem Druck auf die Haut gesetzt. Anschließend wird die Auslöselasche 7 gedrückt und dadurch die Verbindung zwischen der Auslösehülse 6 und dem Gehäuse gelöst. Das Abtriebsglied 10 wird nun durch den Druck des Antriebselement 5 im Gehäuse vorgeschoben. Durch die Kopplung wird die vorschiebende Kraft des Abtriebsglieds 10 von der Nutwandung 12 auf den Ring 21 und von diesem auf die Schulter 22 und somit auf das Übertragungsglied 20 übertragen. Das Übertragungsglied 20 drückt mit einer vorderen Stirnfläche gegen das Behältnis B und den Behältnishalter 30, die unter dem Andruck des Übertragungsglieds 20, von etwaigen Wandreibungskräften abgesehen, zunächst widerstandslos im Gehäuse vorgeschoben werden. Die Nadel N, die exakt in Vorschubrichtung weist, wird über den vorderen Rand des Nadelschutzes 1 hinaus vorgestoßen und dringt in das Gewebe ein. Das Vorschieben des Behältnisses B und damit die Einstichtiefe der Nadel N werden durch Anschlagen der Schulterfläche 32 des Behältnishalters 30 gegen die gehäuseseitige Gegenfläche begrenzt. Das Behältnis B nimmt jetzt seine vordere Position ein.

Während der Vorschubbewegung von Behältnishalter 30 und Behältnis B besteht die Kopplung zwischen dem Abriebsglied 10 und dem Übertragungsglied 20. Die Kopplung ist so ausgelegt, daß sie sich löst, wenn die vordere Position des Behältnisses B erreicht ist.

Dies geschieht durch die Dimensionierung des nachgiebigen Rings 21 und Formgebung der als Anlage für den Ring 21 dienenden Schulter 22. Die Schulter 22 weist in Vorschubrichtung schräg oder bogenförmig vom weiteren hinteren zum engeren vorderen Querschnitt des Übertragungsglieds 20.

Der nachgiebige Ring 21 ist im Querschnitt rund. Grundsätzlich sind die Querschnittsform des Rings 21 und der Verlauf der Schulter 22 lediglich derart aufeinander abzustimmen, daß der nachgiebige Ring 21 die Schulter 22 nicht bereits vor Erreichen der vorderen Behältnisposition überschiebt, wozu die Kopplung zumindest die beim Vorschieben des Behältnisses B auftretende Reaktionskraft und die zum Einstechen der Nadel erforderliche Kraft übertragen muß, und daß die Kopplung nach Erreichen der vorderen Behältnisposition nicht blockiert, sondern sicher löst. Optimal ist die Abstimmung der Kopplung dann, wenn auch das Lösen soweit als möglich ruckfrei erfolgt.

Ist die durch die Kopplungsmittel 12, 21 und 22 gebildete Kopplung gelöst, oder auch bereits während des Lösens, so gelangt das unter dem Druck der Antriebselements 5 weiter vorschiebende Antriebsglied 10 in Andruck gegen die Kolbenstange und wird dadurch mit dem Kolben gekoppelt, der jetzt unter dem Andruck des Abtriebsglieds 10 im Behältnis B vorgeschoben wird, so daß das Produkt verdrängt und durch die Nadel N hindurch ausgeschüttet wird.

Um die mit dem Vorschieben des Abtriebsglieds 10 einhergehende Abnahme der Kraft des Antriebselements 5 zu kompensieren, kann das Übertragungsglied 20 an seiner inneren Mantelfläche konisch aufweitend ausgebildet sein. Der Ring 12 würde bei solcher Ausbildung eine im Verlauf des Verschiebens des Abtriebsglieds 10 abnehmende Reibungskraft erfahren. Dieser Weiterbildung der Erfindung durch kontrollierte Dämpfung der Antriebskraft käme die Elastizität des Rings 21 oder eines gleichwirkenden Andruckmittels zugute.

Alternativ zu dieser auf Steuerung von Gleitreibungskräften beruhenden Kompensation kann die Abnahme der Antriebskraft auch pneumatisch kompensiert werden. Bei pneumatischer Kompensation würde der zwischen den Mantelflächen des Abtriebsglieds 10 und des Übertragungsglieds 20 umschlossene Raum soweit als möglich luftdicht abgeschlossen. Der am rückwärtigen Ende des Übertragungsglieds 20 eingesetzte Führungsring 18 wäre als Dichtring zum Abtriebsglied 10 auszubilden, das selbst einen Kolben bilden würde. Ferner können die zwischen dem Innenmantel des Übertragungsglieds 20 und der gegenüberliegenden Fläche des Stempels 11 gebildeten Gleitflächen als Dichtflächen ausgebildet sein. Auf diese Weise würde beim Vorschieben des Abtriebsglieds 10 ein sich vergrößernder Hohlraum zwischen der äußeren Mantelfläche des Abtriebsglieds 10 und der inneren Mantelfläche des Übertragungsglieds 20 gebildet.

In dem dann als Dichtung ausgebildeten Führungsring 18 wäre lediglich eine kalibrierte Durchgangsöffnung vorzusehen, durch die hindurch ein Medium, vorzugsweise Luft, in den ansonsten dichten Hohlraum nachströmen kann. Der durch das Vorschieben des Abtriebsglieds in diesem Hohlraum erzeugte Unterdruck würde nur verzögert ausgeglichen werden, so daß die Vorschiebegeschwindigkeit des Abtriebsglieds 10 über den gesamten Verschiebeweg des Kolbens K konstant bliebe.

Die Feder 33 bewirkt eine Abfederung und damit Verzögerung der Vorschubbewegung des Behältnishalters 30 mit dem Behältnis B. Allerdings legen der Behältnishalter 30 und das Behältnis B zu Beginn ihrer Vorschubbewegung ein von der Feder 33 nicht behindertes Wegstück zurück, da die Schulter 31 des Behältnishalters 30 erst nach Zurücklegen dieses vorgegebenen Wegstücks in Andruck zur Feder 33 gelangt. Die Vorschubbewegung über den Restweg bis zur vorderen Behältnisposition wird dann durch die im Vergleich zur Kraft des Antriebselements 5 geringere Rückstellkraft der Feder 33 verlangsamt. Hierdurch wird wunschgemäß ein rasches Eindringen der Nadel mit anschließend verlangsamter Vorstoßgeschwindigkeit in die tieferen Gewebeschichten erzielt. An der vorderen Gehäusehülse 2 ausgebildete Anschlaglaschen 2a halten den Behältnishalter 30 in seiner hinteren Stellung. Unter dem Andruck des Antriebselements 5 und infolgedessen des Behältnishälters 30 im Falle des Auslösens geben die Anschlaglaschen 2a jedoch nach. Mittels eines das Gehäuse durchragenden Spanngriffs 7' werden die verschiebbar im Gehäuse aufgenommene Halte- und Auslösehülse 6 und damit zusammen das Abtriebsglied 10 wieder in ihre hintere Stellung gebracht bzw. gespannt. In der hinteren Stellung kann ein neues Behältnis B eingesetzt werden. Das Abtriebsglied 10 und die Hülse 6 sind entsprechend miteinander verbunden.

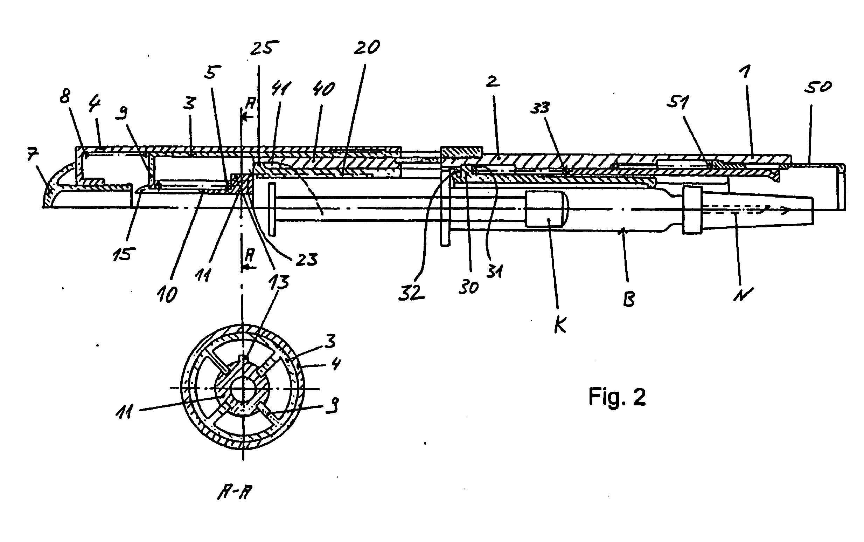

Fig. 2 zeigt eine nicht erfindungsgemäße Ausführungsform eines Autoinjektionsgeräts wiederum in Form eines Pen in einem Längsschnitt und in einem Querschnitt A-A. Bei diesem Pen wird die Antriebsverbindung zwischen dem Abtriebsglied und dem Behältnis sowie dem Abtriebsglied und dem Kolben rein formschlüssig hergestellt und gelöst. Er weist als weiteren Unterschied eine Nadelschutzvorrichtung auf, die die vorgestoßene Nadel nach dem Herausziehen aus dem Gewebe abdeckt und dadurch die Verletzungsgefahr bei der weiteren Handhabung verringert.

Das Gehäuse wird ebenfalls durch eine vordere Gehäusehülse 2 und eine hintere Gehäusehülse 3 gebildet. Der Nadelschutz 1 ist in dieser Ausführungsform einstückig an der vorderen Gehäusehülse 2 ausgebildet. Im hohlzylindrischen Gehäuse ist wieder ein Behältnishalter 30, in dem ein mit dem zu verabreichenden Produkt gefülltes Behältnis B zentriert gehalten ist, entlang der Gehäuselängsachse hin- und her gleitverschiebbar gelagert. Beim Vorschieben zum Einstechen der Nadel N wird der Behältnishalter 30 mit einer vorderen Anschlagfläche 31 gegen einen gehäuseseitigen Anschlag gefahren, wodurch die vordere Position des Behältnisses B bzw. die Einstichtiefe der Nadel N definiert ist. Nach erfolgter Injektion werden die beiden Gehäusehülsen 2 und 3 auseinandergeschraubt, wodurch das Behältnis B und der Behältnishalter 30 von dem Übertragungsglied 20 freikommen und der Behältnishalter 30 unter dem Andruck des als Rückstellfeder ausgebildeten Rückstellelements 33 mit einer hinteren Anschlagfläche 31 gegen eine gehäuseseitige Anschlagfläche in seine hintere Position, die Ausgangsposition, zurückgeschoben wird. In dieser hinteren Position kann ein neues Behältnis B eingesetzt werden. Nach Zusammenschrauben der beiden Gehäusehülsen 2 und 3 ist der Pen der Fig. 2 bereit für die nächste Injektion.

Wie bereits im Ausführungsbeispiel der Fig. 1 wirkt das Abtriebsglied 10 des Pens der Fig. 2 über ein Übertragungsglied 20 auf das Behältnis; ebenso könnte das Übertragungsglied 20 auf den Behältnishalter 30 wirken, der dann das Behältnis B mitnähme. Der Vorschub des Kolbens K im Behältnis B erfolgt durch unmittelbaren Andruck des Stempels 11 des Abtriebsglieds 10 auf die aus dem Behältnis B nach hinten hinausragende Kolbenstange, die am Kolben K von dessen Rückseite abragend angebracht oder einstückig mit dem Kolben K ausgebildet ist.

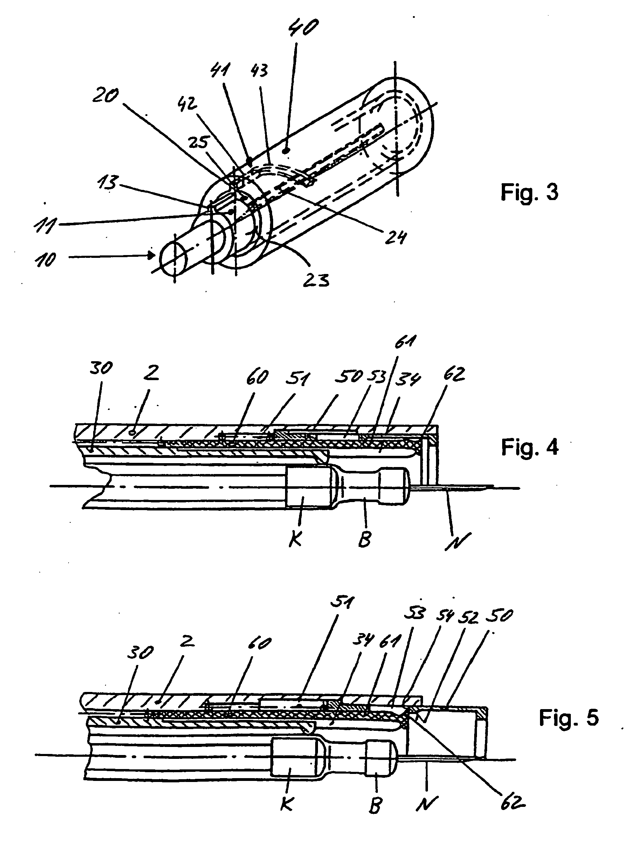

In Fig. 3 sind das Abtriebsglied 10, das Übertragungsglied 20 und ein Führungsmittel 40 entsprechend ihrer Stellung in Fig. 2 dargestellt. In Bezug auf das Koppeln und Entkoppeln des Abtriebsglieds 10 mit und vom Übertragungsglied 20 sei stets auch auf die Darstellung in Fig. 3 verwiesen.

Das Abtriebsglied 10 wird durch einen vollzylindrischen Grundkörper mit einem Stempel 11 mit verbreitertem Querschnitt gebildet. Das Antriebselement 5 ist wiederum als Druckfeder ausgebildet und umgibt ein Zylinderschaftteil des Abtriebsglieds 10. Es ist in der Ausgangsstellung des Pens zwischen dem verdickten Stempel 11 und Rippen 9 eingespannt, die von der hinteren Gehäusehülse 3 radial nach innen auf das Zylinderschaftteil zu ragen. Das Abtriebsglied 10 wird durch Schnapper 15 in seiner Ausgangsstellung gehalten.

Mehrere Schnapper 15 ragen in Verlängerung des Zylinderschaftteils des Antriebsglieds 10 von dem Zylinderschaftteil weg und hintergreifen die radial nach innen ragenden Rippen 9.

Von der äußeren Mantelfläche des Stempels 11 ragt eine Rippe oder ein Nocken 13 ab. Mit dem Nocken 13 liegt das Abtriebsglied 10 auf Anschlag gegen eine hintere Stirnfläche 23 des Übertragungsglieds 20. Das Übertragungsglied 20 wird durch eine einfach hohlzylindrische Hülse, vorzugsweise eine kreiszylindrische Hülse, gebildet, von deren äußeren Mantelfläche in einem hinteren Bereich ein Nocken 25 radial nach außen abragt. An der inneren Mantelfläche des Übertragungsglieds 20 ist eine sich in Längsrichtung des Übertragungsglieds 20 erstreckende, in die Stirnfläche 23 mündende Freigabenut 24 ausgenommen. Die Nut 24 ist zumindest so groß, daß der Nocken 13 des Abriebsglieds 10 in ihr aufgenommen werden kann.

Das Übertragungsglied 20 ist in einer im wesentlichen ebenfalls einfach hohlzylindrischen, vorzugsweise kreiszylindrischen Hülse längsverschiebbar und um die Längsachse drehbar angeordnet. Diese Hülse bildet ein Führungsmittel 40 für das Übertragungsglied 20, dergestalt, daß eine Verschiebebewegung des Übertragungsglieds 20 relativ zum Führungsmittel 40 zwangsweise in eine Drehbewegung des Übertragungsglieds 20 um seine Längsachse relativ zum Führungsmittel 40 umgewandelt wird, d.h. der Verschiebebewegung wird eine Drehbewegung überlagert. Das Führungsmittel 40 ist als eigenständige Hülse ausgebildet, die im Gehäuse verdreh- und verschiebefest aufgenommen ist. Es könnte grundsätzlich auch mit dem Gehäuse, hier mit der hinteren Gehäusehülse 3, einstückig ausgebildet sein, was jedoch die Fertigung aufwendiger gestalten würde.

Die Relativdrehung des Übertragungsglieds 20 gegenüber dem Führungsmittel 40 und dem im Gehäuse verdrehsicher geradgeführten Abtriebsglied 10 wird mittels einer zwischen dem Übertragungsglied 20 und dem Führungsmittel 40 ausgebildeten Kulissenführung bewirkt.

In Ausbildung der Kulissenführung ist an der inneren Mantelfläche des Führungsmittels 40 dem Nocken 25 des Übertragungsgliedes 20 gegenüberliegend eine Führungsnut 41 für diesen Nocken 25 ausgenommen. Die Führungsnut 41 weist einen hinteren geraden Nutabschnitt 42 und einen diesen bogenförmig weiterführenden, gegebenenfalls einfach schräg auslaufenden zweiten Nutabschnitt 43 auf, d.h. der zweite Nutabschnitt 43 weist eine Querkomponente zur Vorschubrichtung des Übertragungsglieds 20 auf. In der Ausgangsstellung kommt der Nocken 25 im Nutabschnitt 42 zu liegen.

Ein Auslösemechanismus weist eine äußere Hülse 4, einen Auslöseknopf 7 und eine Rückstellfeder 8 auf. Die äußere Hülse 4 umgibt den hinteren Bereich der hinteren Gehäusehülse 3 und ist auf der äußeren Mantelfläche der hinteren Gehäusehülse 3 entlang deren Längsachse hin- und her verschiebbar. Der Auslöseknopf 7 ist in eine zentrale Öffnung der hinteren Stirnfläche der äußeren Hülse 4 eingesteckt. Er durchragt diese Öffnung mit einer bis nahe zu den Schnappern 15 des Abtriebsglieds 10 sich erstreckenden Innenhülse.

Zum Injizieren wird der Pen gegen die Haut gedrückt, so daß eine im vorderen Bereich der vorderen Gehäusehülse 2 in Längsrichtung hin- und her verschiebbare Nadelschutzhülse 50 gegen den Druck eines als Rückstellfeder ausgebildeten Rückstellelements 51 in die vordere Gehäusehülse 2 unter den Nadelschutz 1 hineingeschoben wird. In diesem Zustand steht die Nadel N noch hinter dem vorderen Rand, gebildet durch die vorderen Enden des Nadelschutzes 1 und der Nadelschutzhülse 50, zurück.

Das Einstechen der Nadel N und die Ausschüttung des Produkts werden durch Drücken des Auslöseknopfs 7 in Längsrichtung des Pens auf das Abtriebsglied 10 bzw. dessen Schnapper 15 zu bewirkt. Die Innenhülse des Auslöseknopfs 7 überschiebt dabei die hinteren Enden der Schnapper 15, die hierdurch elastisch radial nach innen aufeinander zu gebogen werden, wodurch die Rastverbindung mit den gehäuseseitigen Rippen 9 gelöst wird.

Der Andruck des Antriebselements 5 wird von dem freigegebenen Abtriebsglied 10, das mit seinem Nocken 13 gegen die hintere Stirnfläche 23 des Übertragungsglieds 20 drückt, auf das Übertragungsglied 20 übertragen, das wiederum mit seiner vorderen Stirnfläche gegen eine hintere Flanschfläche des Behältnisses B drückt. Mit der vorderen Stirnfläche des gleichen Flansches drückt das Behältnis B auf den Behältnishalter 30, der seinerseits mittels des Rückstellelements 33 gegen eine dem Gehäuse gegenüber nicht verschiebbare Sperrhülse 60 drückt. Die Druckkraft des Rückstellelemnts 33 ist jedoch gegenüber derjenigen des Antriebselements 5 vergleichsweise gering, so daß unter dem Andruck des Antriebselements 5 das Abtriebsglied 10, das Übertragungsglied 20 und der Behältnishalter 30 mit dem Behältnis B entlang der Gehäuselängsachse im Gehäuse vorgeschoben werden.

Dabei gleitet der Nocken 25 des Übertragungsglieds 20 in seiner Führungsnut 41. Sobald der Nocken 25 bei seinem Vorschieben in den zweiten Nutabschnitt 43 einfährt, wird das Übertragungsglied 20 zwangweise wegen der Querkomponente des zweiten Nutabschnitts 43 in eine Drehung um seine Längsachse gezwungen. Der Verlauf der Führungsnut 41 ist so gewählt, daß durch die erzwungene Relativdrehung gegenüber dem Gehäuse und gegenüber dem Abtriebsglied 10 die im Übertragungsglied 20 ausgenommene Freigabenut 24, die in der Stirnfläche 23 ausmündet, in Überdeckung zu dem Nocken 13 zu liegen kommt.

Sobald die Freigabenut 24 den Nocken 13 überdeckt, ist das Abtriebsglied 10 vom Übertragungsglied 20 entkoppelt. Der Verlauf der Führungsnut 41 ist so gewählt, daß die Überdeckung hergestellt ist, sobald der Behältnishalter 30 mit seiner vorderen Anschlagfläche 31 am Gehäuse auf Anschlag liegt und somit das Behältnis B seine vordere Position einnimmt. Mit der Entkopplung bzw. Freigabe kann das Abtriebsglied 10 dem Übertragungsglied 20 gegenüber unter dem Druck des Antriebselements 5 weiter vorschieben. Es drückt dabei gegen die Kolbenstange, so daß der Kolben K im Behältnis B unter dem unmittelbaren Andruck des Abtriebsglieds 10 vorgeschoben wird, bis das Produkt ausgeschüttet ist.

Das Einstechen der Nadel N wird durch der Verlauf der Führungsnut 41 wunschgemäß festgelegt. Indem sie in einem ersten Teil der Vorschubbewegung des Übertragungsglieds gerade in Vorschubrichtung verläuft und daher dem Vorschieben des Übertragungsglieds 20 kein Widerstand entgegengesetzt wird, sticht die Nadel N zunächst sehr rasch ein. Die Eindringsgeschwindigkeit wird beim weiteren Eindringen verlangsamt, da die Führungsnut 41 dann auch quer zur Vorschubrichtung, im Ausführungsbeispiel wendelförmig, verläuft und demgemäß das Übertragungsglied 20 im Führungsabschriitt 43 beim Vorschieben eine von der Steigung des Führungsabschnitts 43 abhängige Reibungskraft erfährt. Zusätzlich wird die Geschwindigkeit, mit der die Nadel einsticht, durch die zunehmende Rückstellkraft des Rückstellelements 33 verringert.

Die Figuren 4 und 5 zeigen zusammen mit Fig. 2 die Wirkungsweise der Nadelschutzvorrichtung, die verhindert, daß die Nadel N nach dem Herausziehen aus dem Gewebe frei aus dem Gehäuse hervorsteht und abgebrochen werden sowie Verletzungen bei unachtsamer Handhabung verursachen kann. Das wesentliche Merkmal der Nadelschutzvorrichtung ist, daß die zum Zwecke des Einstechens dem Gehäuse gegenüber verschiebbare Nadelschutzhülse 50 nach dem Herausziehen der Nadel in einer Nadelschutzstellung blockiert ist, so daß sie nicht mehr ins Gehäuse geschoben werden kann; ein außenseitiges Überschieben des Gehäuses wäre ebenso denkbar.

Fig. 4 zeigt den vorderen Teil des Pens der Fig. 2 in der vorderen Position des Behältisses B. Die Nadelschutzhülse 50 ist gegen die Kraft des Rückstellelements 51 in ihre hinterste Verschiebeposition der Gehäusehülse 2 gegenüber gedrückt worden. Die Nadel N ragt um die gewünschte Länge aus dem Gehäuse und der Nadelschutzhülse 50 vor.

Die Nadelschutzhülse 50 weist eine hintere Anschlagfläche und eine vordere Anschlagfläche auf, die den Verschiebeweg der Nadelschutzhülse 50 gegenüber der vorderen Gehäusehülse 2 in Vorschubrichtung und gegen die Vorschubrichtung begrenzen. Beim Hin- und Herschieben fährt die Nadelschutzhülse 50 über die Sperrhülse 60, die an ihrem vorderen Ende einen schräg oder bogenförmig nach außen abragenden Haken 62 aufweist. Die Nadel schutzhülse 50 weist in einem Innenmantelbereich, mit dem sie über den Haken 62 gleitet, der Länge ihres maximalen Verschiebewegs in etwa entsprechend, einen leicht verbreiterten Innendurchmesser auf. Ein Übergangsbereich 52 zwischen diesem verbreiterten vorderen Innenquerschnitt zum anschließenden Innenquerschnitt ist abgeschrägt, so daß die Nadelschutzhülse 50 unter dem Andruck des Rückstellelements 51 bis hinter den Übergangsbereich 52 über den Haken 62 gleiten kann. Die Nadelschutzhülse 50 ist hinter dem Übergangsbereich 52 in einem mittleren Bereich mit Längsschlitzen 53 versehen, deren vordere Stirnflächen 54, wie am besten in Fig. 5 zu erkennen ist, Anschlagflächen für je einen der Haken 62 bilden.

Die Sperrhülse 60 läuft in mehreren gleichmäßig über den Hülsenumfang verteilt angeordneten elastisch nachgiebigen Zungen 61 aus, an deren freien vorderen Enden die Haken 62 ausgebildet sind. Der Behältnishalter 30 läuft zu seinem freien vorderen Ende hin ebenfalls in Zungen 34 aus. Durch Vorschieben des Behältnishalters 30 kommen dessen Zungen 34 unter die Zungen 61 der Sperrhülse 60 zu liegen. Jede der Zungen 61 wird so radial nach innen abgestützt und kann in der vorderen Behältnisposition nicht mehr radial einwärts gebogen werden. Die Zungen 61 werden von den Zungen 34 nicht nur abgestützt, sondern zusätzlich radial nach außen gedrückt. Die Zungen 34 sind verglichen mit den Zungen 61 starr ausgebildet oder können in erster Näherung diesen Zungen 61 gegenüber als starr angenommen werden.

Nach dem Herausziehen der Nadel wird die Nadelschützhülse 50 durch das Rückstellelement 51 wieder nach vom geschoben. Sie überschiebt wegen des schrägen Verlaufs der Fläche 52 und/oder des entsprechenden Schrägverlaufs des Hakens 62 diesen Haken 62, dessen auslaufende Spitze zudem elastisch nachgiebig ist. Sobald jedoch die Nadelschutzhülse 50 soweit wieder vorgeschoben worden ist, daß ihre Anschlagfläche 54 in Vorschubrichtung gesehen vor den Haken 62 zu liegen kommt, wird sie durch den gegen die Anschlagfläche 54 auf Anschlag liegenden Haken 62 gegen ein Zurückschieben gesperrt. Der Haken 62 und die Nadelschützhülse 50 liegen mit senkrecht zur Verschieberichtung weisenden Anschlagflächen aneinander. Die Nadel N wird in der in Figur 5 gezeigten Sicherheitsstellung nach der Injektion durch die Nadelschutzhülse 50 geschützt.

Der Behältnishalter 30 wird somit gleichzeitig auch als verschiebbare Unterstützung für das elastische Sperrmittel 62 genutzt und erfüllt nach der Erfindung die Doppelfunktion des Halterns des Behältnisses und des Sperrens der Nadelschutzhülse 50. Die Nadelschutzvorrichtung setzt nicht die erfindungsgemäße Ausbildung des Autoinjektionsgeräts voraus, obgleich sie besonders bevorzugt in Kombination damit Verwendung findet. Sie ist ebenso mit Vorteil bei gattungsgemäßen Autoinjektionsgeräten und sogar unverändert auch bei Injektionsgeräten verwendbar, bei denen das Einstechen der Nadel durch Vorschieben des Behältnisses dem Gehäuse gegenüber manuell erfolgt.

Figur 6 zeigt im Längsschnitt und darüber in einer Draufsicht einen Autoinjektionspen, bei dem die Kopplung des Abtriebsglieds 10 mit dem Behältnis B sowie dem Kolben K auf Kraft- und Formschluß beruht. Die Kopplung ist in ihrer Wirkung der des Pens von Fig. 1 vergleichbar. Im folgenden sei stets auch auf die in der Draufsicht eingetragenen Querschnitte und das Detail X verwiesen.

Bei dem Behältnis B des Pens der Fig. 6 handelt es sich um eine sogenannte Doppelkammerampulle, in der zwei Produktkomponenten in zwei hintereinander angeordneten Kammern, die im Auslieferungszustand des Behältnisses B durch einen vorderen Kolben K1 voneinander getrennt sind, erst durch Vorschieben eines hinteren Kolbens K2 bis gegen den vorderen Kolben K1 miteinander gemischt werden. In der im Auslieferungszustand vorderen Kammer befindet sich im allgemeinen ein pulveriger Wirkstoff, während in der zwischen den beiden Kolben K1 und K2 gebildeten Kammer eine Trägerflüssigkeit aufbewahrt wird.

In dem in Fig. 6 dargestellten Zustand sind die beiden Komponenten bereits durch Vorschieben des hinteren Kolbens K2 bis gegen den vorderen Kolben K1 vollständig in der zwischen dem Kolben K1 und dem Behältnisauslaß liegenden vorderen Kammer durchmischt. Das Gehäuse weist wieder eine vordere und eine hintere Gehäusehülse 2 und 3 auf, die fest miteinander verbunden sind, beispielsweise durch Verschraubung oder Aufstecken. Eine Abdeckkappe 4 ist in die hintere Gehäusehülse 3 eingesteckt. Die vordere Gehäusehülse 1 wird nach vom durch einen Nadelschuz 1 und eine Nadelschutzhülse 50' verlängert, die zum Einstechen der Nadel N gegen eine rückstellende Kraft zurückverschiebbar ist und nach der Injektion wieder die vorgeschobene Stellung einnimmt.

Das Behältnis B ist in einem Behältnishalter 30 bis auf Anschlag eingeschoben und zentriert gehalten. Der Behältnishalter 30 ist gegen die Kraft des Rückstellelements 33 im Gehäuse vorschiebbar.

Das Vorschieben des hinteren Kolbens K2 wird beim Zusammensetzen der vorderen Gehäusehülse 2 und der hinteren Gehäusehülse 3 bewirkt. Zu diesem Zweck ist ein als Hülsenkörper ausgebildetes Mischglied 20 in der hinteren Gehäusehülse 3 verdrehgesichert aufgenommen. Das Mischglied 20 weist einen vorderen Hülsenbereich mit einem Außendurchmesser, der kleiner als der Innendurchmesser des Behältnisses B ist, und einen gegenüber diesem vorderen Hülsenbereich verbreiterten hinteren Hülsenbereich auf. Ein Übergang zwischen diesen beiden Hülsenbereichen ist als radial vom vorderen Hülsebereich abragende Schulter 28 ausgebildet. Die Schulter 28 ist umlaufend ausgebildet; sie kann jedoch auch durch wenigstens einen radial abragenden Steg gebildet werden. Mit seiner hinteren Stirnfläche stößt das Mischglied 20 gegen radial von der hinteren Gehäusehülse 3 nach innen ragende Stege 9, die auch als umlaufende Wandung ausgebildet sein können, an. Beim Zusammensetzen des Geräts, d.h. beim Verschrauben der beiden Gehäusehülsen 2 und 3, wird das derart verschiebegesichert in der hinteren Gehäusehülse 3 aufgenommene Mischglied 20 in das rückwärtig offene Behältnis B eingeführt und darin nach vom geschoben. Dabei drückt es den hinteren Kolben K2 nach vom auf den vorderen Kolben K1 zu bis der hintere Kolben K2 die in Fig. 1 gezeigte Stellung erreicht hat. In dieser Stellung der Kolben K1 und K2 ist auch das Verschrauben der Gehäusehülse 2 und 3 abgeschlossen.

In der hinteren Gehäusehülse 3 ist die Antriebseinrichtung angeordnet, die das Antriebselements 5 in Form einer Druckfeder und das stangenförmige Abtriebsglied 10 umfaßt, das im Gehäuse geradgeführt wird. Das Antriebselement 5 ist zwischen den Stegen 9 und einer den Stegen 9 in Vorschubrichtung gegenüberliegend zugewandten, umlaufenden Schulterfläche des Abtriebsglieds 20 eingespannt.

Das Abtriebsglied 10 ist um eine Mittellängsachse des Gehäuses, die mit seiner eigenen Mittellängsachse zusammenfällt, zwischen zwei Drehstellungen hin- und her verdrehbar aufgenommen. Zum Verdrehen des Abtriebsglieds 20 ist eine das Gehäuse verlängernde Dosierhülse D vorgesehen. In seinem rückwärtigen Bereich, der in die Dosierhülse D hineinragt, weist das Abtriebsglied 10 in Vorschubrichtung sich erstreckende Führungsnuten auf, in die eine in die Abdeckkappe 4 hineinragende, der Abdeckkappe 4 gegenüber verdrehbare, verschiebegesicherte Führungshülse 6a und eine im Gehäuse verdrehbar und verschiebegesichert aufgenommene Anzeigehülse 8 eingreifen. Die Führungshülse 6a ist mit der Dosierhülse D verdrehgesichert verbunden, wie am besten im Schnitt H-H zu erkennen ist. Die Führungshülse 6a dient der Übertragung der Drehbewegung der Dosierhülse D auf das Abtriebsglied 10. Die Anzeigehülse 8, die verdrehgesichert mit dem Abtriebsglied 10 verbunden ist, dient der Anzeige der Verdrehstellung des Abtriebsglieds 10 und damit der Anzeige der eingestellten Dosismenge. Sie ist zu diesem Zweck an ihrem äußeren Umfang mit Markierungen versehen, im Ausführungsbeispiel mit zwei Markierungen für je eine von zwei Drehendstellungen des Abtriebsglieds 10. Die Markierungen können durch eine Gehäuseöffnung hindurch abgelesen werden. Die Anzeigehülse 8 und auch die Führungshülse 6a dienen zusammen mit dem Mischglied 20 als Geradführung für das Abtriebsglied 10.

Das Abtriebsglied 10 wird in der dargestellten hinteren Ausgangsstellung durch eine Sperr- und Auslösemechanik gehalten. Die Sperr- und Auslösmechanik weist ein Auslösemittel 7a in Form eines Auslöseknopfs auf, der quer zur Verschieberichtung des Abtriebsglieds 10 auf ein Sperrmittel 7b wirkt. Der Aufbau und die Wirkungsweise der Sperr- und Auslösemechanik ist am besten in der Zusammenschau des Längsschnitts und des Querschnitts F-F zu erkennen.

Das Sperrmittel 7b wird durch einen Hülsenkörper gebildet mit einer Durchgangsöffnung, die von dem Abtriebsglied 10 durchragt wird. Das Sperrmittel 7b wird zum Zwecke seiner Geradführung quer zur Vorschub- und Längsrichtung des Abtriebsglieds 10 zwischen zwei geraden Stegen des Gehäuses geführt. Diesen beiden Gehäusestegen jeweils zugewandt weist der Hülsenkörper des Sperrmittels 7b dementsprechend gerade Außenflächen auf. Die Durchgangsöffnung des Sperrmittels 7b ist größer als der Außendurchmesser des durchragenden Abtriebsglieds 10. Durch Druck auf das Auslösemittel 7a wird das Sperrmittel 7b gegen die Kraft eines als Druckfeder ausgebildeten Rückstellelements 7c quer zur Vorschubrichtung des Abtriebsglieds 10 verschoben. In der Sperrstellung stößt das Abtriebsglied 10 mit einer durch eine Verdickung gebildete Schulter 29 gegen eine hintere Stirnfläche des Sperrmittels 7b. Durch eine Querverlagerung des Sperrmittels 7b wird dieser Anschlag gelöst. Das Abtriebsglied 10 kommt von dem Sperrmittel 7b frei und kann unter dem Andruck des Antriebselements 5 in Längsrichtung vorschieben.

Eine Sicherheitseinrichtung sorgt dafür, daß das Auslösemittel 7a nur dann betätigt und dadurch das Abtriebsglied 10 freigegeben werden kann, wenn ein Behältnis B in das Gehäuse eingelegt worden ist. Die Sicherheitseinrichtung umfaßt einen Auslösesperrkörper 70 und eine Druckfeder 19. Der Auslösesperrkörper 70 weist einen mittleren Hülsenbereich auf, von dem nach vorne in Längsrichtung zwei Stege 70a abragen (Schnitt E-E). Die beiden Stege 70a durchragen zwei entsprechend geformte Schlitze in der Schulter 28 des Mischglieds 10 und stoßen an den hinteren Rand des Behältnisses B an. Vom mittleren Hülsenbereich des Auslösesperrkörpers 70 ragt ein dritter Steg 70b in Längsrichtung nach hinten ab. Dieser dritte Steg 70b durchragt das Auslösemittel 7a, wie am besten in der Zusammenschau des Längsschnitts mit den beiden Schnitten F-F und G-G zu erkennen ist.

Auf der Höhe des Auslösemittels 7a, d.h. in dem das Auslösemittel 7a durchragenden Bereich, weist der dritte Steg 70b des Auslösesperrkörpers 70 einen Längsschlitz auf. In diesen Längsschlitz fährt eine radial nach innen abragende Rippe 7d des Auslösemittels 7a bei Betätigung des Auslösemittels 7a ein, wenn der Schlitz des Auslösesperrkörpers 70 auf gleicher Höhe wie die Innenrippe 7d des Auslösemittels 7a ist. In Längsrichtung gesehen hinter dem geschlitzten Bereich ist der dritte Steg 70b wieder als geschlossener Steg augebildet. Zwischen dem gehäuseseitigen Steg 9 und einer von der inneren Mantelfläche des mittleren Hülsenbereichs des Auslösesperrkörpers 70 nach innen vorragenden Schulter ist die Druckfeder 19 gespannt. Ist ein Behältnis B eingesetzt, so drückt der Auslösesperrkörper 70 mit seinen vorderen Stegen 70a gegen den hinteren Rand des Behältnisses B und wird so in der im Längsschnitt der Figur 1 dargestellten Stellung gehalten, in der die Innenrippe 7d des Auslösemittels 7a in den Schlitz des Auslösesperrkörpers 70 einfahren und das Sperrmittel 7b querverschieben kann. Falls ein Behältnis nicht eingesetzt worden ist, wird der Auslösesperrkörper 70 durch die Druckfeder 19 in den deshalb dann freien Ringspalt vorgeschoben, bis der mittlere Hülsenbereich des Auslösesperrkörpers 70 gegen die Schulter 28 des Mischglieds 10 stößt. In dieser Sperrstellung des Auslösersperrkörpers 70 kommt der hintere, geschlossene Bereich des dritten Stegs 70b vor die Innenrippe 7d des Auslösemittels 7a zu liegen. Ein Querverschieben des Auslösemittels 7a ist diesem Fall nicht möglich. Die Antriebseinrichtung ist dann gesperrt.

Zwischen dem Mischglied 20 und dem das Mischglied 20 durchragenden Abtriebsglied 10 besteht eine Antriebskopplung, die bewirkt, daß das Mischglied 20 bei einem Vorschieben des Abtriebsglieds 10 vom Abtriebsglied 10 mitgenommen, d.h. selbst relativ zum Gehäuse vorgeschoben wird. Die Kopplung wird durch eine auf Form- und Kraftschluß beruhende Verbindung im vorderen Bereich des Mischglieds 20 hergestellt, die der Kopplung des Ausführungsbeispiels der Figur 1 gleicht. Das Mischglied 20 übernimmt in Doppelfunktion die Funktion eines Übertragungsglieds und wird dementsprechend im folgenden als Misch- und Übertragungsglied 20 bezeichnet.

Im Unterschied zur Kopplung im Ausführungsbeispiel der Fig. 1 ist in dem der Fig. 6 der federelastische Ring 21 in einer am Innenmantel des Misch- und Übertragungsglieds 20 ausgebildeten Nut aufgenommen, und das Abtriebsglied 10 weist dementsprechend lediglich eine die Mitnahme des Misch- und Übertragungsglieds 20 bewirkende Andruckfläche 17 für den Ring 21 auf. Die Andruckfläche 17 bildet ein erstes Kopplungsmittel, eine durch die Nut des Misch- und Übertragungsglieds 20 gebildete Andruckfläche 27 ist ein zweites und der Ring 21 ein drittes Kopplungsmittel.

Das Misch- und Übertragungsglied 20 und das Abtriebsglied 10 bilden in einem innerhalb des Behältnisses B angeordneten Bereich eine Dosiereinrichtung. Das Misch- und Übertragungsglied 20 ist hierfür in einem hinter dem ersten Kopplungsmittel 17 befindlichen Innenmantelbereich mit einer Ausnehmung versehen. Die Ausnehmung weist zwei sich in Vorschubrichtung erstreckende Nuten 25 und 26 auf, die winkelversetzt parallel nebeneinander angeordnet sind. Die Nuten 25 und 26 sind in Vorschubrichtung unterschiedlich lang. Die küizere Nut 25 ist als Sacknut in der Mantelfläche ausgebildet, und die längere Nut 26 wird in Vorschubrichtung durch den hinteren Steg der Aufnahme für das dritte Kopplungsmittel 21 begrenzt. An Ihren rückwärtigen Enden münden die Nuten 25 und 26 auf gleicher Höhe in Bezug auf die Vorschubrichtung in einer Verbreiterung der Ausnehmung aus, wie am besten in Zusammenschau des Längsschnitts mit den Schnitten C-C, D-D und E-E zu erkennen ist. Die Verbreiterung der Ausnehmung reicht bis zur hinteren Stirnfläche des vorderen Hülsenbereichs des Misch- und Übertragungsglieds 20. Die dort auslaufenden, einander gegenüberliegend zugewandten Seitenwände der Verbreiterung werden je durch eine der beiden Nuten 25 und 26 in Vorschubrichtung verlängert.

Das Abtriebsglied 10 ist mit einem radial abragenden Nocken 16 versehen. Im Ausgangszustand des Injektionsgeräts greift der Nocken 16 in die Verbreiterung der Ausnehmung des Misch- und Übertragungsglieds 20 ein. Die Ausnehmung mit den beiden Nuten 25 und 26 bildet ein erstes Dosiermittel und der Nocken 16 ein zweites Dosiermittel der Dosiereinrichtung.

In einer ersten Dosierstellung liegt der Nocken 16 in Flucht zur Nut 25 an der in Vorschubrichtung sich erstreckenden ersten Seitenwand der Verbreiterung an, und in der zweiten Dosierstellung liegt der Nocken 16 fluchtend zur Nut 26 an der in Vorschubrichtung sich erstreckenden zweiten Seitenwand der Verbreiterung der Ausnehmung an. Zwischen diesen beiden Dosierstellungen ist das Abtriebsglied 10 im Ausgangszustand des Injektionsgeräts hin- und her um seine Längsachse verdrehbar. Die beiden Seitenwände der breiten Nut definieren die beiden Dreh- und Dosierstellungen des Abtriebsglieds 10, und die Längen der beiden schmalen Nuten 25 und 26 definieren die Menge der bei einer Injektion ausschüttbaren Wirkstofflösung.

Die Verbreiterung der Ausnehmung im Misch- und Übertragungsglied 20 könnte auch bis zum Ende der kurzen Nut 25 in Vorschubrichtung verlängert werden, so daß die Ausnehmung in Vorschubrichtung einen einfach treppenförmigen Verlauf aufweisen würde.

Zum Durchführen einer Autoinjektion wird das Autoinjektionsgerät mit der vorderen, dem Gehäuse bzw. dem vorderen Nadelschutz 1 gegenüber zurückschiebbaren Nadelschutzhülse 50' auf eine Gewebeoberfläche, insbesondere die menschliche Haut, aufgesetzt. Durch Druck gegen die Gewebeoberfläche wird die Nadelschutzhülse 50' bis in eine hinterste Position relativ zum Gehäuse zurückgeschoben. Die Injektionsnadel N, die am Auslaß am des Behältnisses B in Vorschubrichtung weisend fest angebracht ist, wird zunächst noch von dem Nadelschutz 1 und der darübergeschobenen Nadelschutzhülse 50' bis über ihre vordere Spitze hinaus umgeben und berührt somit noch nicht die Gewebeoberfläche.

Zur Betätigung, d.h. zum Einstechen der Nadel und zur Ausschüttung der Wirkstofflösung, drückt der Verwender, nachdem er das Abtriebsglied 10 mittels der Dosierhülse D in die gewünschte Dreh- bzw. Dosierstellung gebracht hat, das Auslösemittel 7a in radialer Richtung einwärts. Durch das Einwärtsdrücken wird das Sperrmittel 7b unter der Anschlagschulter 29 weggeschoben, und das Abtriebsglied 10 kommt frei. Unter dem Andruck des Antriebselements 5 wird das Abtriebsglied 10 und mittels der Kopplung auch das Misch- und Übertragungsglied 20 relativ zum Gehäuse vorgeschoben. Der Form- und Kraftschluß zwischen dem Abtriebsglied 10 und dem Misch- und Übertragungsglied 20 ist ausreichend stark, um die Mitnahme des Misch- und Übertragungsglieds 10 zu bewirken, das seinerseits mittels seiner Schulter 28 gegen den Behältnishalter 30 und das Behältnis B drückend diese relativ zum Gehäuse und entgegen der rückstellenden Kraft des Rückstellements 33 bis in eine vordere Position vorschiebt, die durch einen gehäuseseitigen Anschlag 31a definiert wird.

In der vorderen Position des Behältnishalters 30 bzw. des Behältnisses B löst die Kopplung die Antriebsverbindung zwischen dem Abtriebsglied 10 und dem Misch- und Übertragungsglied 20. Unter dem weiterhin bestehenden Andruck des Antriebselements 5 wird wegen der Fixierung des Misch- und Übertragungsglieds 20 der nachgiebige Ring 21 zusammengedrückt und über die schräge Schulter 17 geschoben. Das Abtriebsglied 10 schiebt nun auch relativ zum Misch- und Übertragungsglied 20 weiter vor und drückt dabei die beiden Kolben K1 und K2 im Behältnis in Richtung auf den Behältnisauslaß zu ebenfalls vor. Wirkstofflösung wird durch die in der vorderen Position des Behältnisses B in das Gewebe eingestochene Nadel N hindurch ausgeschüttet.

Die Vorschubbewegung des Abtriebsglieds 20 wird in der ersten Dosierstellung durch das vordere Ende der Nut 25 begrenzt. In der ersten Dosierstellung ist die Ausschüttung beendet, wenn der Nocken 16 an diese in Umfangsrichtung sich erstreckende Nutwand anstößt.

## Patentansprüche

1. Autoinjektionsgerät zur Verabreichung eines Produkts, wenigstens umfassend:
a) ein Gehäuse (1, 2, 3),
b) ein vom Gehäuse (1, 2, 3) verschiebbar aufgenommenes Behältnis (B), aus dem durch Vorschieben eines Kolbens (K) Produkt durch eine Nadel (N) an einem Auslaß des Behältnisses (B) ausgeschüttet wird,
c) ein vom Gehäuse (1, 2, 3) verschiebbar aufgenommenes Abtriebsglied (10) einer Antriebseinrichtung, das bei einer Autoinjektion zum Einstechen der Nadel (N) das Behältnis (B) dem Gehäuse (1, 2, 3) gegenüber bis zu einer vorgegebenen vorderen Position und zum Ausschütten von Produkt den Kolben (K) im Behältnis (B) vorschiebt,
d) und eine Kopplung (12, 21, 22; 17, 21, 27), die das Abtriebsglied (10) bis zum Erreichen der vorderen Position des Behältnisses (B) vom Kolben (K) entkoppelt und bei Erreichen dieser Position vom Behältnis (B) entkoppelt
e) wobei das Abtriebsglied (10) bei Erreichen der vorderen Position des Behältnisses (B) zum Vorschieben des Kolbens (K) im Behältnis (B) mit dem Kolben (K) gekoppelt wird.
**dadurch gekennzeichnet, daß**
f) die Kopplung (12, 21, 22; 17, 21, 27) eine kraftschlüssige Verbindung zwischen dem Abtriebsglied (10) und dem Behältnis (B) zumindest umfaßt.

2. Vorrichtung nach Anspruch 1, **dadurch gekennzeichnet, daß** das Abtriebsglied (10) bei dem Vorschieben unmittelbar auf das Behältnis (B) wirkt.

3. Vorrichtung nach Anspruch 1, **dadurch gekennzeichnet, daß** das Abtriebsglied (10) bei einem Vorschieben ein das Behältnis (B) in Vorschubrichtung drückendes Übertragungsglied (20) mitnimmt, daß die die Mitnahme bewirkende Kopplung (12, 21, 22; 13, 23; 17, 21, 27) zwischen dem Abtriebsglied (10) und dem Übertragungsglied (20) gelöst wird, wenn das Behältnis (B) seine vordere Position erreicht und daß das Abtriebsglied (10) bei seinem weiteren Vorschieben gegen den Kolben (K) drückend diesen im Behältnis (B) vorschiebt.

4. Vorrichtung nach einem der vorhergehenden Ansprüche, **dadurch gekennzeichnet, daß** die kraftschlüssige Verbindung zwischen dem Abtriebsglied (10) und einem gegen das Behältnis (B) drückenden Übertragungsglied (20) besteht.

5. Vorrichtung nach dem vorhergehenden Anspruch, **dadurch gekennzeichnet, daß** die Kopplung ein mit dem Abtriebsglied (10) verbundenes erstes Kopplungsmittel (12; 17) und ein mit dem Übertragungsglied (20) verbundenes zweites Kopplungsmittel (22; 27) aufweist, das mit dem ersten Kopplungsmittel (12; 17) unmittelbar order über ein drittes Kopplungsmittel (21) in einem die Mitnahme des Übertragungsglieds (20) bewirkenden Eingriff steht, wobei wenigstens eines dieser Kopplungsmittel nachgiebig ausgebildet ist und unter dem Einfluß einer am Ende der Vorschubewegung des Behältnisses (B) auftretenden Kraft nachgibt und **dadurch** der Eingriff gelöst wird.

6. Vorrichtung nach dem vorhergehenden Anspruch, **dadurch gekennzeichnet, daß** das dritte Kopplungsmittel (21) ein nachgiebiger Ring ist, der einen in Vorschubrichtung wirkenden Druck des ersten Kopplungsmittels (12; 17) auf das zweite Kopplungsmittel (22; 27) überträgt und bei Überschreiten einer in Vorschubrichtung wirkenden, vorgegebenen Maximalkraft über das erste Kopplungsmittel (12; 17) oder das zweite Kopplungsmittel (22; 27) hinaus verschoben wird.

7. Vorrichtung nach einem der Ansprüche 3 bis 7, **dadurch gekennzeichnet, daß** zur Kompensation einer mit dem Vorschieben des Abtriebsglieds (10) einhergehenden Abnahme einer das Vorschieben bewirkenden Antriebskraft eine zwischen dem Übertragungsglied (20) und dem relativ dazu vorschiebenden Abtriebsglied (10) erzeugte Dämpfung ebenfalls abnimmt.

8. Vorrichtung nach einem der Ansprüche 3 bis 8, **dadurch gekennzeichnet, daß** das Behältnis (B) durch eine Mehrkammerampulle gebildet wird und ein Mischglied, insbesondere ein Mischrohr, zum Vorschieben eines hinteren Kolbens (K2) in Richtung auf einen vorderen Kolben (K1) das Übertragungsglied (20) bildet.

9. Vorrichtung nach dem vorhergehenden Anspruch, **dadurch gekennzeichnet, daß** das Übertragungsglied (20) wenigstens ein Dosiermittel (25, 26) aufweist, das im Zusammenwirken mit der Antriebseinrichtung die Auswahl einer zu verabreichenden Produktdosis ermöglicht.

10. Vorrichtung nach einem der vorhergehenden Ansprüche, **dadurch gekennzeichnet, daß** eine am Gehäuse (1, 2, 3) verschiebbar angebrachte Nadelschutzhülse (50) bei vorgeschobener Nadel (N) in einer die Nadel (N) umhüllenden vorderen Stellung gegen ein Zurückschieben dem Gehäuse (1, 2, 3) gegenüber gesperrt wird.

11. Vorrichtung nach dem vorhergehenden Anspruch, **dadurch gekennzeichnet, daß** ein vom Gehäuse (1, 2, 3) verschiebbar aufgenommener Behältnishalter (30), der mit dem Behältnis (B) vorgeschoben wird, in der vorderen Position des Behältnisses (B) die Sperrung der Nadelschutzhülse (50) bewirkt.

12. Vorrichtung nach einem der Ansprüche 3 bis 8, **dadurch gekennzeichnet, daß** das Übertragungsglied ein Behältnishalter ist.

13. Vorrichtung nach einem der Ansprüche 1 bis 5, 8 bis 10 und 13, **dadurch gekennzeichnet, daß** die Kopplung ein oder mehrere elastisch nachgiebige Schnapper umfaßt und daß der oder die Schnapper unmittelbar am Abtriebsglied ausgebildet ist oder sind, das damit gegen eine Andruckfläche des Behältnisses oder des optionalen Übertragungsglieds drückt, oder daß der oder die Schnapper am optionalen Übertragungsglied vorgesehen ist oder sind.

## Claims

1. An auto-injection device for administering a product, comprising at least:
a) a housing (1, 2, 3);
b) a container (B), shiftably accommodated by the housing (1, 2, 3), from which product is dispensed through a needle (N) at an outlet of the container (B) by advancing a piston (K);
c) a driven member (10) of a drive unit, shiftably accommodated by the housing (1, 2, 3), which - during an auto-injection for injecting the needle (N) - advances the container (B) to a predetermined frontal position in relation to the housing (1, 2, 3) and advances the piston (K) within the container (B) for dispensing product;
d) and a coupling (12, 21, 22; 17, 21, 27) which decouples the driven member (10) from the piston (K) until the container (B) reaches the frontal position and decouples the driven member (10) from the container (B) once this position is reached,
e) wherein, when the container (B) reaches the frontal position, the driven member (10) is coupled to the piston (K) in order to advance the piston (K) within the container (B);
**characterised in that**
f) the coupling (12, 21, 22; 17, 21, 27) comprises at least a non-positive locking connection between the driven member (10) and the container (B).

2. The device according to claim 1, **characterised in that** when advancing, the driven member (10) acts directly on the container (B).

3. The device according to claim 1, **characterised in that**: when advancing, the driven member (10) slaves a transfer member (20) which pushes the container (B) in a forward direction; the coupling (12, 21, 22; 13, 23; 17, 21, 27) between the driven member (10) and the transfer member (20) which effects the slaving is released when the container (B) reaches its frontal position; and **in that**, when the driven member (10) advances further, it pushes against the piston (K), advancing it within the container (B).

4. The device according to any one of the preceding claims, **characterised in that** the non-positive locking connection exists between the driven member (10) and a transfer member (20) pressing against the container (B).

5. The device according to the preceding claim, **characterised in that** the coupling comprises a first coupling means (12; 17) connected to the driven member (10) and a second coupling means (22; 27) connected to the transfer member (20), the second coupling means (22; 27) engaging with the first coupling means (12; 17) directly or via a third coupling means (21), said engagement slaving the transfer member (20), and wherein at least one of said coupling means is formed flexible and yields under the influence of a force exerted at the end of the forward movement of the container (B), thus releasing the engagement.

6. The device according to the preceding claim, **characterised in that** the third coupling means (21) is a flexible ring which transmits a pressure of the first coupling means (12; 17) acting in the forward direction onto the second coupling means (22; 27) and is shifted beyond the first coupling means (12; 17) or the second coupling means (22; 27) when a predetermined maximum force acting in the forward direction is exceeded.

7. The device according to any one of claims 3 to 7, **characterised in that** in order to compensate for a decrease in a drive force which effects the advancing, said decrease being associated with advancing the driven member (10), a dampening generated between the transfer member (20) and the driven member (10) advancing relative to it likewise decreases.

8. The device according to any one of claims 3 to 8, **characterised in that** the container (B) is formed by a multi-chamber ampoule, and a mixing member - in particular a mixing tube - for advancing a rear piston (K2) towards a frontal piston (K1) forms the transfer member (20).

9. The device according to the preceding claim, **characterised in that** the transfer member (20) comprises at least a dosing means (25, 26) which in co-operation with the drive unit allows a product dose which is to be administered to be selected.

10. The device according to any one of the preceding claims, **characterised in that** when the needle (N) is advanced, a needle safety sleeve (50) which is shiftably arranged on the housing (1, 2, 3) is blocked - in a frontal position surrounding the needle (N) - against retracting relative to the housing (1, 2, 3).

11. The device according to the preceding claim, **characterised in that** a container holder (30), shiftably accommodated by the housing (1, 2, 3) and advanced together with the container (B), blocks the needle safety sleeve (50) when the container (B) is in its frontal position.

12. The device according to any one of claims 3 to 8, **characterised in that** the transfer member is a container holder.

13. The device according to any one of claims 1 to 5, 8 to 10 and 13, **characterised in that** the coupling comprises one or more elastically flexible snap mechanisms and **in that** the snap mechanism or snap mechanisms is/are directly formed on the driven member which uses them to push against a stop face on the container or optional transfer member, or **in that** the snap mechanism or snap mechanisms is/are arranged on the transfer member.

## Revendications

1. Dispositif automatique d'injection pour l'administration d'un produit, comprenant au moins :
a) un boîtier (1, 2, 3),
b) un conteneur (B) logé par le boîtier de manière coulissable, hors duquel du produit est expulsé par l'avance d'un piston (K) à travers une aiguille (N) située à une sortie du conteneur (B),
c) un membre entraîné (10) d'un dispositif d'entraînement logé par le boîtier (1, 2, 3) de manière coulissable, membre qui pousse, lors d'une injection automatique en vue de piquer l'aiguille (N), le conteneur (B) face au boîtier (1, 2, 3) jusqu'à une position avant prescrite et le piston (K) dans le conteneur (B) pour expulser le produit,
d) et un accouplement (12, 21, 22 ; 17, 21, 27), qui découple le membre entraîné (10) jusqu'à ce que la position avant du conteneur (B) soit atteinte par le piston (K) et le découple lorsque le conteneur (B) a atteint cette position,
e) le membre entraîné (10) étant couplé, lorsque le conteneur (B) a atteint la position avant, pour pousser le piston (K) dans le conteneur (B) avec le piston (K).
**caractérisé en ce que**
f) l'accouplement (12, 21, 22 ; 17, 21, 27) comprend au moins une liaison par concordance de force entre le membre entraîné (10) et le conteneur (B).

2. Dispositif selon la revendication 1, **caractérisé en ce que** le membre entraîné (10) agit directement sur le conteneur (B) lors de l'avance.

3. Dispositif selon la revendication 1, **caractérisé en ce que** le membre entraîné (10) entraîne avec lui, lors d'une avance, un membre de transmission (20) poussant le conteneur (B) en direction d'avance, **en ce que** l'accouplement exerçant l'entraînement (12, 21, 22 ; 17, 21, 27) est débloqué entre le membre entraîné (10) et le membre de transmission (20), lorsque le conteneur (B) atteint sa position avant et **en ce que** le membre entraîné (10) avance lors de son autre avance, en poussant contre le piston (K), celui-ci dans le conteneur (B).

4. Dispositif selon l'une quelconque des revendications précédentes, **caractérisé en ce qu'**il y a liaison par concordance de force entre le membre entraîné (10) et un membre de transmission (20) poussant contre le conteneur (B).

5. Dispositif selon l'une quelconque des revendications précédentes, **caractérisé en ce que** l'accouplement présente un premier moyen d'accouplement (12 ; 17) relié au membre entraîné (10) et un deuxième moyen d'accouplement (22 ; 27) relié au membre de transmission (20), qui est dans une relation de pénétration exerçant l'entraînement du membre de transmission (20), avec le premier moyen d'accouplement (12 ; 17) directement ou par l'intermédiaire d'un troisième moyen d'accouplement (21), un de ces moyens d'accouplement étant au moins conçu de manière souple et fléchissant sous l'influence d'une force intervenant à la fin du mouvement d'avance du conteneur (B) et déclenchant ainsi la pénétration.

6. Dispositif selon l'une quelconque des revendications précédentes, **caractérisé en ce que** le troisième moyen d'accouplement (21) est un anneau souple qui transmet une pression s'exerçant en direction d'avance du premier moyen d'accouplement (12 ;17) sur le deuxième moyen d'accouplement (22 ; 27) et qui est poussé vers l'extérieur lors du dépassement d'une force maximale prescrite, s'exerçant en direction d'avance, par l'intermédiaire du premier moyen d'accouplement (12 ; 17) ou du deuxième moyen d'accouplement (22 ; 27).

7. Dispositif selon l'une quelconque des revendications 3 à 7, **caractérisé en ce que** pour la compensation d'une réduction accompagnant l'avance du membre entraîné (10) d'une force d'entraînement exerçant l'avance, un amortissement produit entre le membre de transmission (20) et le membre entraîné (10) poussant de manière relative décroît également.

8. Dispositif selon l'une quelconque des revendications 3 à 8, **caractérisé en ce que** le conteneur (B) est constitué d'une ampoule à plusieurs chambres et qu'un membre mélangeur, en particulier un tube mélangeur, pour faire avancer un piston arrière (K2) en direction d'un piston avant (K1) constitue le membre de transmission (20).

9. Dispositif selon l'une quelconque des revendications précédentes, **caractérisé en ce que** le membre de transmission (20) présente au moins un moyen de dosage (25, 26), qui permet dans l'action conjointe avec le dispositif d'entraînement, la sélection d'une dose de produit à administrer.

10. Dispositif selon l'une quelconque des revendications précédentes, **caractérisé en ce qu'**une enveloppe de protection d'aiguille (50) montée sur le boîtier (1, 2, 3) de manière coulissable est bloquée lorsque l'aiguille (N) s'avance dans une position avant enveloppant l'aiguille (N) pour empêcher un retour par rapport au boîtier (1, 2, 3).

11. Dispositif selon l'une quelconque des revendications précédentes, **caractérisé en ce qu'**un support de conteneur (30) logé de manière coulissable par le boîtier (1, 2, 3), qui est avancé avec le conteneur (B), exerce en position avant du conteneur (B) le blocage de l'enveloppe de protection de l'aiguille (50).

12. Dispositif selon l'une quelconque des revendications 3 à 8, **caractérisé en ce que** le membre de transmission est un support de conteneur.

13. Dispositif selon l'une quelconque des revendications 1 à 5, 8 à 10 et 13, **caractérisé en ce que** l'accouplement comprend un ou plusieurs accouplements à déclic souples élastiques et **en ce que** le ou les accouplements à déclic est ou sont conçus directement sur le membre entraîné, qui ainsi pousse contre une surface de pression du conteneur ou du membre de transmission optionnel, ou **en ce que** le ou les accouplements à déclic est ou sont prévus sur le membre de transmission optionnel.
